# EUROPÄISCHE PATENTANMELDUNG

(11) **EP 0 659 442 A1**
(43) Veröffentlichungstag der Anmeldung: **28.06.1995**
(21) Anmeldenummer: 94116545.8
(22) Anmeldetag: 20.10.1994
(51) Int. Cl.: A61L 33/00, C08J 3/20

(54) **Verfahren zur Herstellung von medizinischen Arbeitsmitteln**

(30) Priorität: 07.12.1993 DE 4341628
(71) Anmelder: REHAU AG + Co, 95111 Rehau (DE)
(72) Erfinder: Kühlein, Georg, c/o Rehau AG + Co., D-95111 Rehau (DE)

(57) **Zusammenfassung**

Die Erfindung betrifft ein Verfahren zur Herstellung von medizinischen Arbeitsmitteln, z.B. Katheter, Schläuche, Behälter, Formteile und dergleichen aus polymeren Materialien. Diese Arbeitsmittel weisen eine besondere Ausstattung zur Erhöhung der Blutkompatibilität bei der Berührung mit Blut oder blutähnlichen Flüssigkeiten auf. Das Kennzeichen der Erfindung wird darin gesehen, daß an die einzelnen Granulatteilchen des Polymermaterials vor deren Thermoplastifizierung eine definierte Menge eines Additivs angelagert wird. Dieses Additiv ist in der Polymerschmelze löslich, im Glaszustand jedoch mit dem Polymeren nicht mischbar. Die derart behandelten Granulatteilchen werden anschließend thermoplastifiziert und zu dem Endprodukt verarbeitet.

## Beschreibung

Die Erfindung betrifft ein Verfahren zur Herstellung von medizinischen Arbeitsmitteln wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen.

Aus der DE-A 19 30 136 ist ein beschichteter Katheter bekannt, welcher als Basis aus einem Schlauch aus Gummi oder einem Elastomeren besteht. Dieser Katheter ist an der Außen- und/oder Innenwand mit einem hydrophilen Polyacrylat bzw. -metacrylat, insbesondere mit einem Hydroxi-Alcyl oder einem Hydroxialcoxy-Acrylat bzw. -metacrylat mit jeweils niederen Alcylresten beschichtet.

Weiterhin ist es bekannt, derartige medizinische Arbeitsmittel mit einer Beschichtung aus Heparin zu versehen. Diese Heparin-Beschichtungsverfahren arbeiten über APTES- und TDMAC-Brücken. Mit dieser Heparinbeschichtung wird das Anheften von Blutplättchen an der Oberfläche des medizinischen Arbeitsmittels verhindert und gleichzeitig die intrinsische Koagulation desaktiviert. Ausführungen zum Beschichtungsverfahren mit Heparin über TDMAC-Brücken finden sich bei G.A. Grode, R.D. Falb, J.P. Crowley in J. Biomed.Mater.Res.Symp., 3, 77 (1972). Entsprechende Hinweise zur Beschichtung mit Heparin über APTES-Brücken finden sich bei R.L. Merker, L.J. Elyasch, S.W. Mayhew, J.Y.C. Wang in Proc.Artif.Heart Conf. 1969, Seite 29.

Alle diese Verfahren haben den prinzipiellen Nachteil, daß sie technisch aufwendig und sehr arbeitsintensiv sind. So läuft eine Heparinisierung bei beiden genannten Verfahren in mehreren zusätzlichen Arbeits- und Beschichtungszyklen ab.

Ein weiterer wesentlicher Nachteil dieser bekannten Verfahren ist es, daß die auf diese Weise hergestellten medizinischen Halbzeuge sterilisiert werden müssen, weil die Beschichtung mit Heparin mikrobiell abgebaut werden kann. Da nach der Konfektion der Halbzeuge zu Fertigprodukten eine Gesamtsterilisation erforderlich ist, ist hier als weiterer Nachteil das erhebliche Risiko einer Mehrfachsterilisation zu nennen.

Aus der EP 04 71 364 A 2 ist ferner die Verwendung von zyklischen Estern der Salicylsäure als Vorprodukte zur Herstellung von medizinischen Artikeln bekannt, die in erster Linie bioabsorptionsfähig ausgestattet sind. Aufgrund dieser speziellen Materialeigenschaften können damit Klammern, Knochennägel und ähnliche Artikel hergestellt werden.

Desweiteren ist aus der Fachzeitschrift Münchn.med.Wschr. 130 (1988) Nr.46, Seiten 809-814 die Verwendung von Acetylsalicylsäure als Antithrombotikum bekannt. Diese Veröffentlichung beschäftigt sich mit dem Nutzen der Acetylsalidylsäure für die klinische Prävention.

Schließlich ist es aus der diesseitigen DE 42 21 665 C 1 bekannt, ein medizinisches Arbeitsmittel mit erhöhter Blutkompatibilität herzustellen, bei dem in das Ausgangspolymere vor der eigentlichen Formgebung eine definierte Länge von Acetylsalicylsäureestern bzw. -mischestern bzw. -teilestern als Zuschlagstoffe eingemischt sind. Dabei wird von der Überlegung ausgegangen, daß dem Ausgangspolymeren als Additive Acetylsalicylsäureester bzw. -mischester zugesetzt wird, wobei diese Zuschlagstoffe das Ausgangspolymere mit ihrem relativen Gerinnungsparameter dem Quotienten 1,0 annähern. Beim Arbeiten mit dem Verfahren hat sich herausgestellt, daß die Oberflächen der medizinischen Arbeitsmittel erst nach einem gewissen Zeitablauf ihre optimale Blutkompatibilität erhalten. Dies kann auf die langsame Oberflächenmigration der die Blutkompatibilität bewirkenden Additive aus dem Querschnitt des medizinischen Arbeitsmittels an dessen Oberfläche zurückzuführen sein.

Hier setzt die Erfindung ein, die es sich zur Aufgabe gestellt hat, diesen Migrationsprozess zu beschleunigen und einen Zuschlagstoff anzugeben, der das damit versetzte medizinische Arbeitsmittel unmittelbar nach der Herstellung für den vorgesehenen Gebrauch einsatzfähig macht. Erfindungsgemäß wird dazu vorgeschlagen, daß an die einzelnen Granulatteilchen des Polymermaterials vor deren Thermoplastifizierung eine definierte Menge eines in der Polymerschmelze löslichen, jedoch im Glaszustand mit dem Polymer nicht mischbaren Additivs angelagert wird, und daß die solcherart behandelten Granulatteilchen anschließend thermoplastifiziert und zum Endprodukt verarbeitet werden.

Die Erfindung benutzt hierbei die Unverträglichkeit der chemischen Bestandteile der Polymermischung als sogenannte Gleitschiene, um die an die Polymerpartikel angekoppelten Antikoagulantien an der Oberfläche des medizinischen Arbeitsmittels anzureichern. Die blutkompatible Wirkung bei dem hergestellten Endprodukt wird damit beschleunigt und verstärkt.

Das erfindungsgemäße Additiv ist vorteilhaft ein Amidwachs auf der Basis Carbonsäure (D₂-C₁₆)/Acetylsalicylsäure-Ethylendiamin. Dieses Additiv ist in der Polymerschmelze löslich, jedoch im Glaszustand nicht mit dem Polymer mischbar und in verarbeitetem Zustand migriert es an die Oberfläche des medizinischen Arbeitsmittels. Bei der Arbeit mit dieser Mischung wurde festgestellt, daß sich schon während des Formgebungsprozesses eine geschlossene und gleichmäßige Schicht des Amidwachses auf der Oberfläche des sich im Status Nascendi befindlichen medizinischen Arbeitsmittels bildet. Diese Schicht wird verstärkt durch die nachträglich aus dem Querschnitt des medizinischen Arbeitsmittels weiterhin an die Oberfläche austretenden Amidwachs-Teilchen. Vorteilhaft wird das erfindungsgemäße Additiv in einer Menge von 0,02 bis 1,0 Gewichtsprozent dem Ausgangspolymeren zugemischt. Bei diesem Mischprozess spielen die Verarbeitungsparameter eine entscheidende Rolle.

Durch ein intensives Vermengen von Polymer-Granulat und Amidwachs als Zuschlagstoff und durch ein geeignetes Temperaturprogramm beim Aufschmelzen läßt sich erfindungsgemäß die Ausbildung einer gleichmäßigen Beschichtung schon im Herstellprozess erzielen. Hierzu wird bei der Vermengung des Polymer-Granulats mit dem Amidwachs das Gemenge nur auf eine Temperatur erhitzt, bei der die jeweilige Wachskomponente des Additivs schmilzt. Erst danach wird das Gemenge auf die für die Formgebung des Polymermaterials notwendige Verarbeitungstemperatur gebracht.

Diese Temperaturen liegen bei einem Gemenge von Polyvinylchlorid mit Amidwachs bei 133°C, von Polyurethan mit Selten-Erd-Carboxylat bei 145°C und von Synthetic-Rubber-Thermoplastic (SRT) mit Acetylsalicylsäure-Ethylendiamin bei 80°C.

Es liegt im Rahmen der Erfindung, daß dem Ausgangspolymeren ein zusätzliches Additiv in Form von Selten-Erd-Carboxilaten in einer Menge von 0,02 bis 5 Gewichtsprozent zugemischt wird. Diese Zusatzadditive vermeiden einen koagulationsauslösenden Einfluß der im Polymermaterial für medizinische Einweg-Artike üblicherweise verwendeten Stabilisatoren Kalzium-Stearat und Zink-Stearat. Hierbei ist darauf hinzuweisen, daß die gerinnungshemmende Wirkung solcher zusätzlicher Additive bekannt ist.

Schließlich erscheint es zweckmäßig, daß sowohl das erfindungsgemäße Amidwachs als auch die Selten-Erd-Carboxilate als Ersatz undd/oder Ergänzung weiterer Zuschlagstoffe zum Ausgangspolymeren wie Stabilisatoren, Gleitmittel usw. verwendet werden. Dabei kommen die stabilisierenden und gleitenden Eigenschaften der erfindungsgemäßen Additive zum Einsatz, wodurch die eine Koagulation auslösenden Eigenschaften der weiteren Additive in den verarbeiteten Polymermaterialien vermieden wird.

Bei den mit den erfindungsgemäßen Additiven ausgestatteten medizinischen Arbeitsmitteln lassen sich die Blutkompatibilitätseigenschaften durch Beimengung verschiedener Gewichtsprozent-Anteile der Additive zu dem Ausgangspolymeren variieren.

### Beispiel 1

100 Teile Polyurethan-Rezeptur
1 Teil Selten-Erd-Carboxylat

### Beispiel 2

100 Teile Polyvinylchlorid-Rezeptur
0,2 Teile Acetylsalicylsäure-Ethylendiamine

### Beispiel 3

100 Teile Synthetic Rubber Thermoplastic (SRT)
2,5 Teile Selten-Erd-Carboxylat

## Patentansprüche

1. Verfahren zur Herstellung von medizinischen Arbeitsmitteln wie Katheter, Schläuche, Behälter, Formteile und dergleichen aus polymeren Materialien, wobei diese Arbeitsmittel eine besondere Ausstattung zur Erhöhung der Blutkompatibilität bei der Berührung mit Blut oder blutähnlichen Flüssigkeiten aufweisen, dadurch gekennzeichnet, daß an die einzelnen Granulatteilchen des Polymermaterials vor deren Thermoplastifizierung eine definierte Menge eines in der Polymerschmelze löslichen, jedoch im Glaszustand mit den Polymeren nicht mischbaren Additivs angelagert wird, und daß die solcherart behandelten Granulatteilchen anschließend thermoplastifiziert und zu dem Endprodukt verarbeitet werden.

2. Verfahren nach Anspruch 1, dadurch gekennzeichnet, daß das Additiv ein Amidwachs auf der Basis Karbonsäure (C₂-C₁₆)/Acetylsalicylsäureäthylendiamin ist.

3. Verfahren nach Ansprüchen 1 und 2, dadurch gekennzeichnet, daß das Additiv in einer Menge von 0,02 bis 1,0 Gewichtsprozent dem Ausgangspolymeren zugesetzt wird.

4. Verfahren nach Anspruch 1, dadurch gekennzeichnet, daß dem Ausgangspolymeren ein zusätzliches Additiv in Form von Selten-Erd-Carboxilaten in einer Menge von 0,02 bis 5 Gewichtsprozent zugemischt wird.

5. Verfahren nach Ansprüchen 1 und 4, dadurch gekennzeichnet, daß sowohl das Amidwachs als auch die Selten-Erd-Carboxilate als Ersatz und/oder Ergänzung der weiteren Zuschlagstoffe zum Ausgangspolymeren wie Stabilisatoren, Gleitmittel und dergleichen verwendet werden.
